# EUROPEAN PATENT APPLICATION

(11) **EP 1 736 776 A2**
(43) Date of publication of application: **27.12.2006**
(21) Application number: 06012879.0
(22) Date of filing: 22.06.2006
(51) Int. Cl.: G01N 33/50

(54) **Method and reagent for measuring kinase activity**

(30) Priority: 23.06.2005 JP 2005184093
(71) Applicant: SYSMEX CORPORATION, Kobe-shi, Hyogo 651-0073 (JP)
(72) Inventor: Matsushima, Tomoko, Kobe-shi, Hyogo 651-0073 (JP); Ishihara, Ideki, Kobe-shi, Hyogo 651-0073 (JP)
(74) Representative: HOFFMANN EITLE

(57) **Abstract**

A method of measuring the activity of a kinase to which imatinibmesylate canbind is described. In the method, a kinase contained in a sample, to which imatinib mesylate can bind, a substrate for the kinase, and ATP are mixed. A phosphate group of ATP is thereby transferred to a tyrosine residue of the substrate by the action of the kinase. After a labeled substance capable of binding is bound to the substrate having the phosphate group transferred thereto, a signal emitted by the labeled substance is detected. On the basis of this detection result, the activity of the kinase is calculated.

## Description

### BACKGROUND OF THE INVENTION

### 1. Field of the Invention

The present invention relates to a method of measuring the activity of a kinase to which imatinib mesylate can bind and a reagent therefor.

### 2. Description of the Related Art

An abnormal chromosome called Philadelphia chromosome formed by mutual translocation between chromosomes 9 and 22 can be specifically observed in tumor cells from patients with chronic myeloid leukemia (CML). In the chromosome 9, there is a gene called c-abl which upon occurrence of a break in the vicinity of this gene, is translocated to a place called bcr (breakpoint cluster region) of the chromosome 22. The two genes are thereby fused with each other to form a chimera gene bcr/abl. It is estimated that Bcr/Abl, that is, a kinase encoded by this chimera gene accelerates cell growth signals abnormally to cause abnormal proliferation of leukemic cells, thus inducing CML.

Moreover, it is believed that gastrointestinal stromal tumor (GIST) is induced by mutation of c-kit gene and subsequent excessive action of an enzyme called KIT encoded by this gene.

As an effective method for treating CML and GIST, there is chemotherapy using a molecular target drug Glivec (Novartis Pharma K.K.) using imatinib mesylate as an active ingredient. Imatinib mesylate binds to a kinase such as Bcr/Abl or KIT to inhibit its function thereby exhibiting an antitumor effect on these diseases.

However, Glivec does not have an antitumor effect on all patients with CML, and has a high effect only on patients with CML who have a high activity of Bcr/Abl. Nevertheless, Glivec is administered as a first-line drug to all patients who receive chemotherapy. Under the present circumstance, therefore, Glivec has been administered even to patients for whom the efficacy of Glivec administered is low.

Further, Glivec does not have an antitumor effect on all patients with GIST, and has a high effect only on patients with GIST who have a high activity of KIT. In chemotherapy for GIST, Glivec is administered only to KIT-positive patients with GIST. As a method of judging whether the patient is positive or negative to KIT, there is an immune histological method of staining excised tumor cells with a labeled antibody binding specifically to KIT. In this method, however, false positive may result depending on the skill of a technician. In the case of false positive, Glivec may be administered although the efficacy of Glivec administered is intrinsically low for the patient.

The patient for whom the effect of Glivec administered is low as described above must, upon administration of Glivec, bear physical burdens such as side effect and economical burdens such as therapeutic cost although the efficacy is low. Therefore, there is demand for development of a method or reagent for accurately measuring the activity of kinase targeted by imatinib mesylate as the active ingredient of Glivec for selective administration of Glivec only to patients for whom the effect of Glivec is high.

Le Coutre et al. in Journal of.the National Cancer Institute, 91:163-168, 1999 describe a method of measuring the activity of kinase by using a labeled antibody binding specifically to auto-phosphorylated Bcr/Abl (referred to hereinafter as auto-phosphorylation measurement method) as a method of measuring the activity of Bcr/Abl kinase. This method is based on the fact that Bcr/Abl is activated upon auto-phosphorylation thereby enabling transfer of a phosphate group to a substrate. This method, however, does not measure the actually phosphorylated substrate directly, and thus the degree of auto-phosphorylation of Bcr/Abl does not always agree with the degree of phosphorylation of the substrate by Bcr/Abl. Accordingly, this method cannot accurately measure the activity of kinase targeted by imatinib mesylate.

### SUMMARY OF THE INVENTION

The object of the present invention is to provide a method capable of accurately measuring the activity of a kinase to which imatinib mesylate can bind, as well as a reagent therefor.

A first aspect of the present invention relates to a method for measuring the activity of a kinase to which imatinib mesylate can bind, comprising steps of: transferring a phosphate group of ATP to a substrate for the kinase by contacting the kinase with the substrate and ATP, the kinase contained in a sample; binding a labeled substance to the substrate having the transferred phosphate group; and measuring the activity of the kinase on the basis of the label of the labeled substance bound to the substrate.

A second aspect of the present invention relates to a reagent for measuring the activity of a kinase to which imatinib mesylate can bind, comprising: a substrate for the kinase; ATP; and a labeled substance capable of binding to the substrate having a phosphate group transferred thereto.

### BRIEF DESCRIPTION OF THE DRAWINGS

Fig. 1 is a graph showing the kinase activity of each of measurement samples contained in containers i to v.
Fig. 2 is a graph showing the measurement result of the kinase activity of each of measurement samples A to D.
Fig. 3 is a fluorescence photograph showing the result of blotting in Example 3.
Fig. 4 is a fluorescence photograph showing the result of blotting in Example 4.
Fig. 5 is a graph showing the result of blotting in Fig. 4.

### DESCRIPTION OF THE PREFERRED EMBODIMENTS

According to the method of measuring the activity of kinase in this embodiment, the activity of kinase is measured in the following manner. First, a kinase contained in a sample, to which imatinib mesylate can bind, a substrate for the kinase, and ATP are mixed. A phosphate group of ATP is thereby transferred to a tyrosine residue of the substrate by the action of the kinase. After a labeled substance capable of binding is bound to the substrate having the phosphate group transferred thereto, a signal emitted by the labeled substance is detected. On the basis of this detection result, the activity of the kinase is calculated. According to this method of measuring the activity of kinase, the amount of the substrate phosphorylated by the kinase to which imatinibmesylate can bind can be measured, and thus the activity of the kinase can be accurately measured.

The kinase to be measured for its activity is not particularly limited insofar as it is a kinase to which imatinib mesylate can bind. Specific examples include an enzyme having activated Abl, or KIT. The enzyme having activated Abl can be exemplified by Bcr/Abl. The enzyme having activated Abl, and KIT, are each tyrosine kinase transferring a phosphate group to a tyrosine residue of a polypeptide serving as the substrate.

Imatinib mesylate is a water-soluble powdery substance with a chemical name
4-(4-methylpiperazin-1-ylmethyl)-N-[4-methyl-3-(4-pyridin-3-ylpyrimidin-2-ylamino)phenyl] benzamide monomethanesulfonate. Imatinib mesylate has a property of binding to the site of a specific kinase to which ATP binds (ATP-binding site).

The sample is not particularly limited insofar as it contains a kinase as a subject of measurement, and a biological sample containing tumor cells, tumor cell mass or the like can be used. Further, a liquid sample obtained by solubilizing the biological sample by physical and/or chemical treatment, or a sample obtained by further purifying the liquid sample by centrifugation etc. can also be used.

ATP and the substrate are contacted with the kinase contained in the above sample. When Bcr/Abl for example is measured for activity as the kinase to be measured for activity in this embodiment, the substrate may be any polypeptide capable of binding to Bcr/Abl. Specifically, myelin basic protein (MBP), Grb2 or the like can be used as the substrate, and particularly MBP is preferably used. When the activity of KIT is measured, the substrate used may be any polypeptide capable of binding to KIT, and particularly Grb2 is preferably used.

By contacting ATP and the substrate with the kinase, a tyrosine residue in the substrate is phosphorylated by the action of the kinase. A labeled substance binding specifically to the substrate having the phosphorylated tyrosine residue (referred to hereinafter as phosphorylated substrate) is contacted with, and bound to, the phosphorylated substrate. By detecting the label of the labeled substance bound to the phosphorylated substrate, the activity of the kinase can be measured.

The labeled substance is not particularly limited insofar as it has a substance capable of binding specifically to the phosphorylated substrate and generating a detectable signal (signal-generating substance).

For example, a labeled substance consisting of both the signal-generating substance and an antibody binding specifically to the phosphorylated substrate can be used. In this case, the labeled substance binds to the phosphorylated substrate thereby detecting a signal emitted by the labeled substance, whereby the activity of the kinase can be measured.

As the labeled substance, both an antibody which is capable of binding specifically to the phosphorylated substrate and has avidin or biotin added thereto and a signal-generating substance which has biotin or avidin added thereto can also be used. In this case, the antibody binds to the phosphorylated substrate, and the biotin or avidin added to the signal-generating substance binds to the avidin or biotin added to the antibody. Then, the activity of the kinase is measured by detecting a signal emitted by the signal-generating substance.

As the labeled substance, three materials, that is, a primary antibody which is capable of binding specifically to the phosphorylated substrate, a secondary antibody which is capable of binding specifically to the primary antibody and has avidin or biotin added thereto, and a signal-generating substance which has biotin or avidin added thereto can also be used. In this case, the primary antibody binds to the phosphorylated substrate, and the secondary antibody binds to the primary antibody, and the biotin or avidin of the signal-generating substance binds to the avidin or biotin of the secondary antibody. Then, the activity of the kinase is measured by detecting a signal emitted by the signal-generating substance.

In this specification, "avidin" is intended to encompass streptavidin.

The signal-generating substance may be any substance capable of generating a detectable signal. For example, a radioactive isotope, a fluorescence substance, an enzyme etc. can be used, and a fluorescence substance is preferably used. As the fluorescence substance, Cy3, Cy5, FITC, SYBR GREENII, YOYO, GFP etc. can be used.

When the labeled substance having an antibody is used, the antibody binding specifically to phosphorylated tyrosine in the substrate (referred to hereinafter as phosphorylated tyrosine) is preferably used. As the antibody, an antibody obtained by purification from animal blood, an antibody obtained by genetic recombination, a polyclonal antibody, a monoclonal antibody, and a mixture of two or more thereof can be used. The antibody referred to herein also includes antibody fragments and derivatives thereof. Specific examples include Fab, Fab', F(ab)₂ and sFv fragment (Blazar et al., 1997, Journal of Immunology, 159: 5821-5833 and Bird et al., 1988, Science, 242: 423-426). The subclass of the antibody is not limited to IgG, and antibodies of other subclass, such as IgM, may also be used.

Le Coutre et al. in Journal of the National Cancer Institute, 91:163-168, 1999 describe an auto-phosphorylation measurement method as a method of measuring the activity of Bcr/Abl, but this method does not measure the actually phosphorylated substrate directly, and the degree of auto-phosphorylation of the kinase does not always agree with the degree of phosphorylation of the substrate by the kinase. Accordingly, the measurement method in the present embodiment wherein the phosphorylated substrate is directly detected can measure the activity of the kinase more accurately than the above-mentioned auto-phosphorylation measurement method.

The efficacy of Glivec for the patient can be predicted on the basis of the activity determined by the method of measuring the activity of kinase in this embodiment.

Glivec is an anti-malignant tumor agent comprising imatinib mesylate as a major ingredient, and is confirmed at present to be effective against CML and GIST. Imatinibmesylate is fit into the ATP-binding site of the kinase so that ATP cannot bind to the kinase having imatinib mesylate bound to the ATP-binding site thereof. Glivec thereby inhibits the activity of the kinase, to exhibit an antitumor effect on CML and GIST.

Glivec is effective for those patients who among patients with CML, have a high activity of Bcr/Abl, and also effective for those patients who among patients with GIST, have a high activity of KIT. Accordingly, the activity of the kinase measured by the kinase activity measurement method described above can be an indicator for prediction of the efficacy of administered Glivec on the patients, and Glivec can be administered selectively to only those patients for whom the administration of Glivec was predicted to be effective. When the efficacy of Glivec is to be predicted, a threshold value, for example, may be established and compared with the kinase activity to predict the efficacy. Specifically, the measured activity of the kinase is compared with the corresponding threshold value, and when the activity is not lower than the threshold value, the administration of Glivec is predicted to be effective, while when the activity is lower than the threshold value, the administration of Glivec is predicted to be ineffective. For establishing the threshold value, for example, tumor cells from each of a group of CML patients and a group of GIST patients prior to the administration of Glivec are measured for their Bcr/Abl activity or KIT activity, and Glivec is administered to these patients for a predetermined period to examine its efficacy, and the Bcr/Abl activity or KIT activity is compared with the presence or absence of the efficacy of Glivec, and the activity, at which the above group can be classified in a high proportion into a patient group for whom the administration of Glivec is effective and a patient group for whom the administration of Glivec is ineffective, can be established as the threshold value.

The amount of administered Glivec can be regulated depending on the activity of the kinase in order to conduct therapy adapted to the efficacy in the patient.

As the sample used in predicting the efficacy of Glivec, a biological sample containing tumor cells or tumor cell mass collected from a CML patient or a GIST patient can be used. Further, a liquid sample obtained by solubilizing such biological sample by physical and/or chemical treatment, or a sample obtained by further purifying the liquid sample by centrifugation etc. can also be used.

The reagent for measuring the activity of kinase in this embodiment comprises a substrate for kinase, ATP, and a labeled substance capable of binding to a phosphorylated substrate. These ingredients may be contained in a single container, or at least one of the ingredients may be contained in another container. Preferably, a first reagent containing the substrate and ATP is contained in a first container, and a second reagent containing the labeled substance is contained in a second container. When the labeled substance comprises, for example, the primary antibody, the secondary antibody and the signal-generating substance, these are contained preferably in containers different from one another.

The reagent may contain a buffer solution for buffering the pH. The type of the buffer solution is not particularly limited, and for example, Good's buffer solution, phosphate buffer solution, etc. can be used. When the respective ingredients are contained in a plurality of containers, each container may contain a buffer solution.

### EXAMPLES

### Example 1: Inhibition of Bcr/Abl by imatinib mesylate

### 1. Preparation of a measurement sample

KU812 cells established as a strain from patients with CML were homogenized in IP buffer (containing 0.1% NP-40, 25 mM Tris (pH 7.4), 150 mM NaCl, 25 mM NaF, 0.5 mM vanadate, 1 mM DTT, 100 µg/ml PMSF, 0.2% protease inhibitor) to prepare a lysate. The resulting lysate was prepared such that the protein in the lysate became 50 µg and this sample was used as a measurement sample A and introduced into 5 containers i to v respectively.

### 2. Immune precipitation

The measurement sample A contained in the containers i to v was subjected to immune precipitation in the following manner.

5 µg anti-Bcr antibody, Bcr (N-20):sc-885 (Santa Cruz Biotechnology, Inc.), was bound to 40 µl of 50% protein A beads (Amersham Biosciences). The protein A beads having Bcr (N-20) :sc-885 bound thereto were added to each container, and 20 µl IP buffer was added thereto. Then, each container was stirred gently at 4°C for 1 hour so that Bcr/Abl contained in the measurement sample A contained in each container was captured by the protein A beads.

1 ml IP buffer was further added to each of the containers i to v, and the protein A beads having Bcr/Abl bound thereto were washed by upside-down mixing, and after centrifugation, the supernatant was discharged. 1 ml TS buffer (containing 50 mM Tris, 150 mM NaCl) was added to each container and the protein A beads were washed by upside-down mixing, and after centrifugation, the supernatant was discharged. Further, 1 ml Tris buffer (containing 50 mM Tris) was added to each container and the protein A beads were washed by upside-down mixing, and after centrifugation, the supernatant was discharged.

### 4. Kinase reaction

After the immune precipitation, a reagent for Bcr/Abl reaction containing 40 µg MBP (SIGMA-ALDRICH), 2 mM ATP (Sigma) and a buffer solution (containing 50 mM Tris (pH 7.4), 10 mM MgCl₂, 0.1 mM EDTA, 0.375% Brij 35, 0.1 mg/ml BSA, 1 mM DTT) was added to each container. Further, 0.1 µM imatinib mesylate was added to the container ii, 1 µM imatinib mesylate to the container iii, 10 µM imatinib mesylate to the container iv, and 100 µM imatinib mesylate to the container v. Imatinib mesylate was not added to the container i.

After imatinib mesylate was added, each container was vigorously stirred at 30°C for 30 minutes to effect kinase reaction.

### 5. Detection

The amount of tyrosine residues phosphorylated by the kinase reaction can be indicative of the activity of Bcr/Abl.

After the kinase reaction, TBS was added to the measurement sample A contained in each container such that the total protein contained in the measurement sample A was adjusted to 0.4 µg/50 µl, and 50 µl measurement sample A was added to each well of a slot blotter having a PVDF membrane set thereon. The sample in the well was suctioned at the bottom of the well, that is, at the backside of the PVDF membrane, so that the protein in the measurement sample was adsorbed onto the PVDF membrane. The PVDF membrane was separated from the slot blotter, then shaken for 60 minutes together with 8 ml (1 µg/ml) primary antibody solution and then washed with TBS. Then, the membrane was shaken for 60 minutes together with 8 ml (1 µg/ml) secondary antibody solution.

In the primary antibody solution, an antibody binding specifically to phosphorylated tyrosine, that is, anti-pTyr (4G10) mouse (Upstate, Inc.), was contained as the primary antibody. In the secondary antibody solution, an antibody binding specifically to the primary antibody, that is, biotinylated F(ab')2 fragment of rabbit anti-mouse IgG (Dako Cytomation), was contained as the secondary antibody. Biotin had been added to the secondary antibody.

The PVDF membrane which had been reacted with the primary antibody solution and the secondary antibody solution was washed with TBS and then shaken for 60 minutes together with 8 ml (2 µg/ml) FITC solution containing streptavidin FITC (Vector Laboratories, Inc.), whereby the phosphorylated substrate on the PVDF membrane was labeled with FITC. Thereafter, the PVDF membrane was washed with TBS and further washed with distilled water. After washing, the PVDF membrane was dried at 60°C, and the fluorescence intensity of the phosphorylated substrate adsorbed onto the PVDF membrane was analyzed by an image analyzer (BIO-RAD Laboratories) to determine the fluorescence intensity, and on the basis of a previously prepared calibration curve, the phosphorylated substrate labeled with FITC was quantified.

Hereinafter, a method of preparing the calibration curve is described. First, a rabbit IgG antibody solution was mixed at 5 different concentrations with TBS solutions containing 1 µg/l ml BSA, and then 50 µl each of the resulting mixtures was put to each well previously treated in the same manner as described above. The resulting mixture was labeled with FITC by the same method as described above by using a labeled substance consisting of an antibody binding to the rabbit IgG antibody and FITC, and then measured for its fluorescence intensity, and the relationship between the fluorescence intensity and concentration was graphed to prepare a calibration curve.

### 6. Results

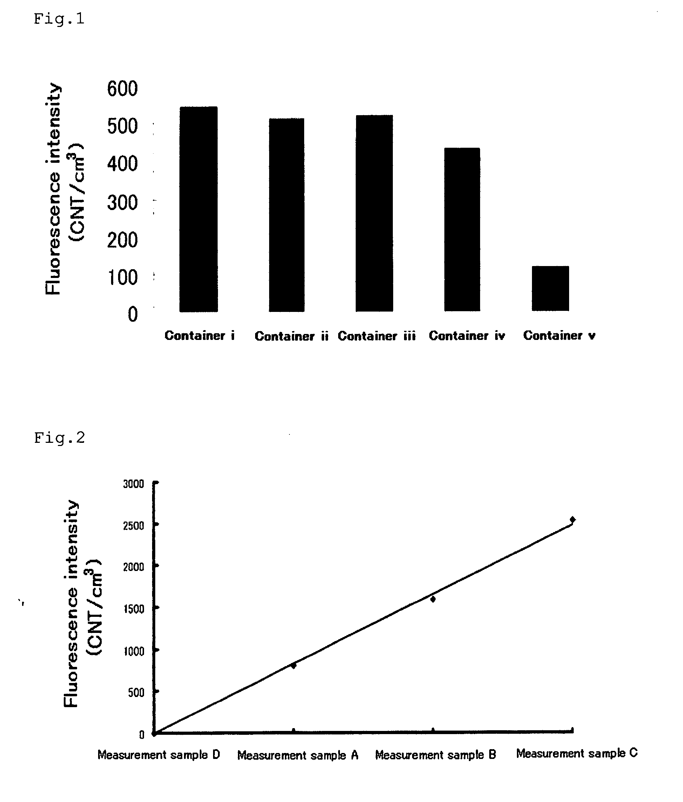

Fig. 1 is a graph showing the kinase activity of each of the measurement samples contained in the containers i to v. Fig. 1 reveals that as the amount of imatinib mesylate added is increased, the kinase activity is decreased. Thus, it was confirmed that the kinase activity measured in the experimental procedure in this example is the activity of Bcr/Abl whose activity is inhibited by imatinib mesylate.

### Example 2: Measurement of the activity of Bcr/Abl

### 1. Preparation of measurement samples

A measurement sample A was prepared in the same manner as Example 1 such that the total protein contained therein became 50 µg. A measurement sample B was prepared such that the total protein contained therein became 100 µg, and a measurement sample C was prepared such that the total protein contained therein became 150 µg. As the negative control, a measurement sample D consisting exclusively of IP buffer was prepared.

### 2. Immune precipitation, kinase reaction, and detection

The measurement samples A to D were subjected to immune precipitation in the same experimental procedure as in Example 1. After the immune precipitation, the measurement samples A to D were subjected to kinase reaction in the same procedure as in Example 1 except that imatinib mesylate was not added. After the kinase reaction, the measurement samples A to D were subjected to detection in the same experimental procedure as in Example 1.

### 3. Results

Fig. 2 is a graph showing the measurement result of the kinase activity of each of the measurement samples A to D. As can be seen from Fig. 2, the activity of Bcr/Abl contained in each of the measurement samples was proportional to the amount of the protein contained in each of the measurement samples, thus showing extremely excellent quantitativeness. It was thus revealed that the activity of Bcr/Abl in each measurement sample could be accurately measured.

### Example 3: Selection of useful substrate for measurement of KIT activity

### 1. Preparation of measurement samples

HEL cells established as a strain from patients with GIST were homogenized in IP buffer to prepare a lysate. The resulting lysate was prepared such that the protein in the lysate became 50 µg, and the resulting sample was used as a measurement sample E and introduced into 3 containers vi to viii respectively.

### 2. Immune precipitation

The measurement sample E contained in the containers vi to viii was subjected to immune precipitation in the same experimental procedure as in Example 1 except that an anti-KIT antibody, that is, C-19 (Santa Cruz Biotechnology, Inc.) was used in place of the anti-Bcr antibody.

### 3. Kinase reaction

After the immune precipitation, 3 kinds of reaction reagents for KIT reaction were prepared and added to the containers vi to viii respectively. An MBP-containing reagent for KIT reaction (containing 4.4 µg MBP, 2 mM ATP, 50 mM Tris (pH 7.4), 10 mM MgCl₂, 10 mM MnCl₂, 0.1 mM EDTA, 0.375% Brij35 and 1 mM DTT) was added to the container vi. A histone H1-containing reagent for KIT reaction (having the same composition as in the MBP-containing reagent for KIT reaction except that 4.4 µg histone H1 was contained in place of 4.4 µg MBP) was added to the container vii. A Grb2-containing reagent for KIT reaction (having the same composition as in the MBP-containing reagent for KIT reaction except that 4.4 µg Grb2 was contained in place of 4.4 µg MBP) was added to the container viii.

After each reagent for KIT reaction was added, each of the containers was vigorously stirred at 30°C for 30 minutes to effect kinase reaction.

### 4. Detection

The measurement sample E contained in the containers vi to viii was subjected to detection in the same manner as in Example 1.

### 5. Results

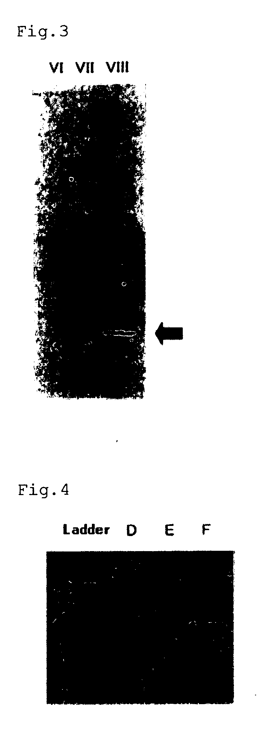

Fig. 3 is a fluorescence photograph showing the result of blotting. In Fig. 3, lane VI is blotting of MBP in the sample contained in the container vi, lane VII is blotting of histone H1 in the sample contained in the container vii, and lane VIII is blotting of Grb2 in the sample contained in the container viii. In Fig. 3, fluorescence was observed only in lane VIII (shown by an arrow in Fig. 3). That is, it appears that only Grb2 was phosphorylated by the action of KIT and labeled with FITC. Accordingly, it was revealed that Grb2 can be used most preferably as the substrate for measuring the activity of KIT.

### Example 4: Measurement of KIT activity by using Grb2

### 1. Preparation of measurement samples

A measurement sample E was prepared in the same manner as Example 3 such that the total protein contained therein became 50 µg. A measurement sample F was also prepared such that the total protein contained therein became 75 µg. As the negative control, a measurement sample D consisting exclusively of IP buffer was prepared.

### 2. Immune precipitation

The measurement samples D to F were subjected to immune precipitation in the same experimental procedure as in Example 3.

### 3. Kinase reaction

After the immune precipitation, 4.4 µg Grb2 was added to each of the measurement samples, and the measurement samples D to F were subjected to kinase reaction in the same experimental procedure as in Example 3.

### 4. Detection

The measurement samples D to F were subj ected to detection in the same experimental procedure as in Example 1.

### 5. Results

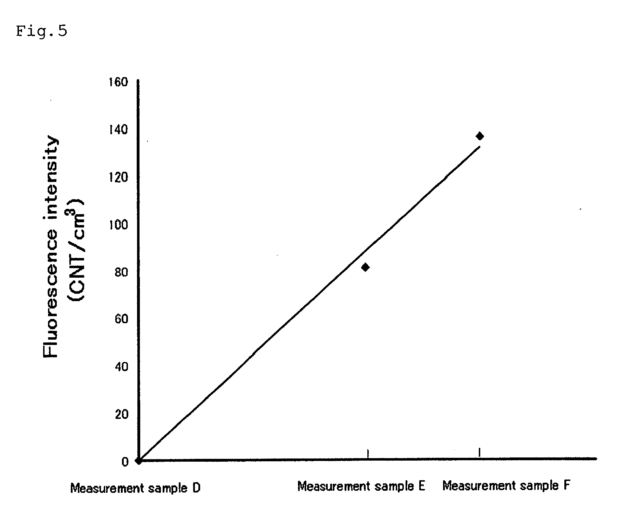

Fig. 4 is a fluorescence photograph showing the result of blotting. In Fig. 4, lane D is blotting of the measurement sample D after kinase reaction, lane E is blotting of the measurement sample E after kinase reaction, and lane F is blotting of the measurement sample F after kinase reaction. The left-most lane is a ladder. Fig. 5 is a graph showing the result of blotting in Fig. 4. In Fig. 5, the activity of KIT contained in each of the measurement samples is proportional to the amount of the protein contained in each of the measurement samples, thus exhibiting extremely excellent quantitativeness. It was thus found that the activity of KIT in each measurement sample could be accurately measured.

## Claims

1. A method for measuring the activity of a kinase to which imatinib mesylate can bind, comprising steps of:
transferring a phosphate group of ATP to a substrate for the kinase by contacting the kinase with the substrate and ATP, the kinase contained in a sample;
binding a labeled substance to the substrate having the transferred phosphate group; and
measuring the activity of the kinase on the basis of the label of the labeled substance bound to the substrate.

2. The method of claim 1, wherein the kinase has activated Abl.

3. The method of claim 2, wherein the kinase is Bcr/Abl.

4. The method of claim 1, wherein the substrate is myelin basic protein.

5. The method of claim 1, wherein the sample is a biological sample collected from a patient with chronic myeloid leukemia.

6. The method of claim 1, wherein the kinase is KIT.

7. The method of claim 1, wherein the substrate is Grb2.

8. The method of claim 1, wherein the sample is a biological sample collected from a patient with gastrointestinal stromal tumor.

9. The method of claim 1, further comprising a step of contacting the sample containing the kinase with an antibody against the kinase to capture the kinase by the antibody,
wherein the kinase of the transferring step is the kinase captured by the antibody.

10. The method of claim 9, wherein the antibody is an anti-Bcr antibody or anti-KIT antibody.

11. A reagent for measuring the activity of a kinase to which imatinib mesylate can bind, comprising:
a substrate for the kinase;
ATP; and
a labeled substance capable of binding to the substrate having a phosphate group transferred thereto.

12. The reagent of claim 11, wherein the labeled substance has an antibody capable of binding to the substrate having a phosphate group transferred thereto, and a signal-generating substance.

13. The reagent of claim 12, wherein the labeled substance comprises:
a primary antibody which is capable of binding to the substrate having a phosphate group transferred thereto;
a secondary antibody which is capable of binding to the primary antibody and has avidin or biotin bound thereto; and
a signal-generating substance which has biotin or avidin bound thereto.

14. The reagent of claim 12, wherein the signal-generating substance is a fluorescence substance.

15. The reagent of claim 11, further comprising an antibody against the kinase.

16. The reagent of claim 15, wherein the antibody is an anti-Bcr antibody or anti-KIT antibody.
